# EUROPEAN PATENT SPECIFICATION

(11) **EP 2 167 955 B1**
(45) Date of publication and mention of the grant of the patent: **03.05.2017**
(21) Application number: 08792868.5
(22) Date of filing: 23.07.2008
(51) Int. Cl.: G01N 33/48, B01L 3/00

(54) **CHIP FOR ANALYZING FLUIDS**
CHIP ZUR ANALYSE VON FLÜSSIGKEITEN
PUCE D'ANALYSE DE FLUIDES

(30) Priority: 23.07.2007 KR 20070073659
(43) Date of publication of application: 31.03.2010
(73) Proprietor: NanoEnTek, Inc., Guro 3-dong, Guro-Gu Seoul 152-711 (KR)
(72) Inventor: PARK, Jun Ha, Suwon-si Gyeonggi-do 440-729 (KR); CHUNG, Chan Il, Seoul 135-866 (KR); CHANG, Jun Keun, Seoul (KR)
(74) Representative: Louis Pöhlau Lohrentz
(86) International application number: PCT/KR2008/004314
(87) International publication number: WO 2009/014379

(56) References cited:
- WO-A1-2005/062059
- WO-A1-2005/119211
- WO-A1-2007/040313
- WO-A1-2008/086809
- FR-A1- 2 790 684

## Description

### Technical Field

The present invention relates to a chip having a microchannel, through which fluids moves, and more particularly to a chip for analyzing sample fluids, which includes a sample inlet and a sample outlet, and has a structure where the sample inlet and the sample outlet communicate with each other through a closed channel with a pipe shape.

### Background Art

In general, biological, chemical, and /or optical analysis of sample fluids is used in analyzing blood clinically collected from a patient and diagnosing diseases, as well as a chemical field and a biotechnology field. Various kinds of chip structures have been developed and used so as to provide an analyzing and/or diagnosing apparatus, which has a further smaller size, and performs analysis of sample fluid in more effective manner. As such, the object of development of a lab-on-a-chip is to make it possible for various functions to be performed in one chip so as to increase effectiveness in analysis and/or diagnosis of diseases, and to make it possible to manufacture a rapid kit.

A lab-on-a-chip means implementing various test processes, which are performed in a laboratory, for example, the separation, refinement, mixing, labeling, analysis, and washing, etc. of a sample, on a chip having a small size. Techniques related to microfluidics and a micro-LHS are typically used in designing the lab-on-a-chip. Also, in manufacturing a chip-structure implementing microfluidics and the micro-LHS, a chip, in which a microchannel is formed at the interior of the chip by using a semiconductor circuit designing technique, has been put on the market.

In general, detecting and analyzing analytes in a very small amount, which are included in sample fluid, such as blood, a body fluid, urine, etc., includes analyzing if a sample fluid reacts against proteins, such as antigens, antibodies, etc. or other material, which have been previously immobilized on a chip, while moving through a channel having a pipe-shaped structure formed in the interior of the chip, through detection of fluorescent material, etc. Therefore, a technique for observing movement of fluids moving in a chip having a channel through the channel and a technique for manufacturing a channel structure are the most important core techniques in manufacturing a small-sized chip for performing fluid analysis and obtaining accurate analysis result by using such a chip.

In a chip (or a structure) having a microchannel implementing microfluidics, in order to allow fluids to flow through an inner space formed by a microchannel, a small-size motor is used, or a method for limiting the width and height of a channel so as to allow fluids to move through a microchannel due to capillary phenomenon is used. At this time, in a chip where main driving force causing movement of fluids is capillary force, the result of investigation shows that fact that fluids flowing in a space formed by a channel have an irregular and nonuniform movement pattern. It is understood that such a phenomenon occurs because acting force due to relative action between upper and lower inner walls of a channel and fluids is different from acting force due to relative action between left and lower inner walls of the channel and fluids. As a result, such a nonuniform movement pattern of fluids has been a big obstacle to detection and analysis of analytes in a very small amount in the fluids.

WO 2005/119211 A1 describes a device for collection blood and separating blood constituents, comprising inter alia a cover and a carrier, a feed device for collecting the blood, and a venting device for venting the sample liquid in the inlet region, wherein the venting device has a recess laterally adjacent to the channel and wherein the venting device has at least one venting channel in the carrier of the device connected to the recess.

FR 2 790 684 A1 discloses an apparatus comprising at least one planar surface where compartments are formed and are defined by a partition, the compartments creating a space which allows for the displacement of a liquid sample or the independent movement of at least two liquid samples, the compartments made up of at least two different types of groove.

Meanwhile, a chip, which has a sample inlet and a sample outlet included at both ends thereof and has a structure where fluids injected into the sample inlet is discharged through the sample outlet through a closed channel shaped similar to a pipe, is manufactured in such a manner that upper and lower substrates are manufactured and are assembled with each other. However, in order to manufacture a microchannel structure having a size below several tens of micrometers, it is difficult to process edge parts of a channel without loss of other part thereof, and it is also difficult to control standards and quality of a product in mass production. Also, the fine difference of such a channel structure obstructs uniform flux of fluids so that it causes a sample analysis result without consistency in a chip used in detecting analytes in an extremely small amount from a small amount of a sample.

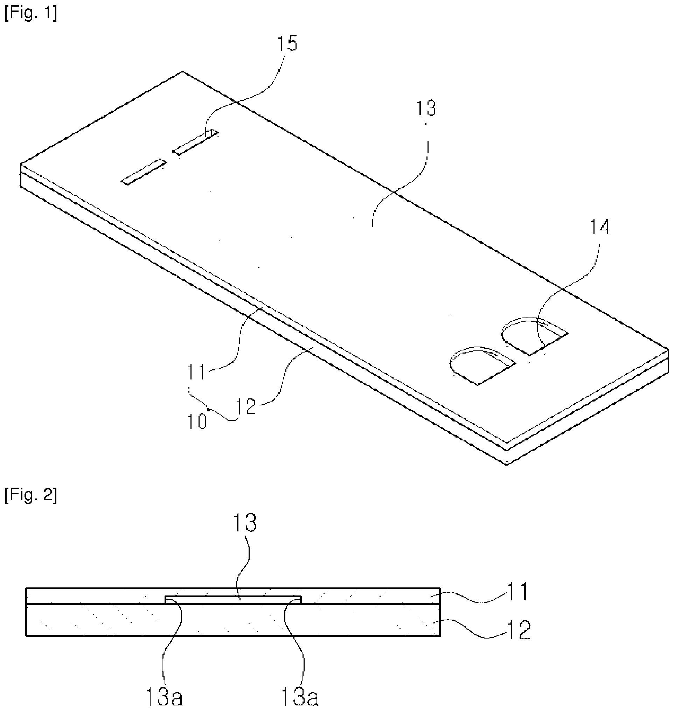

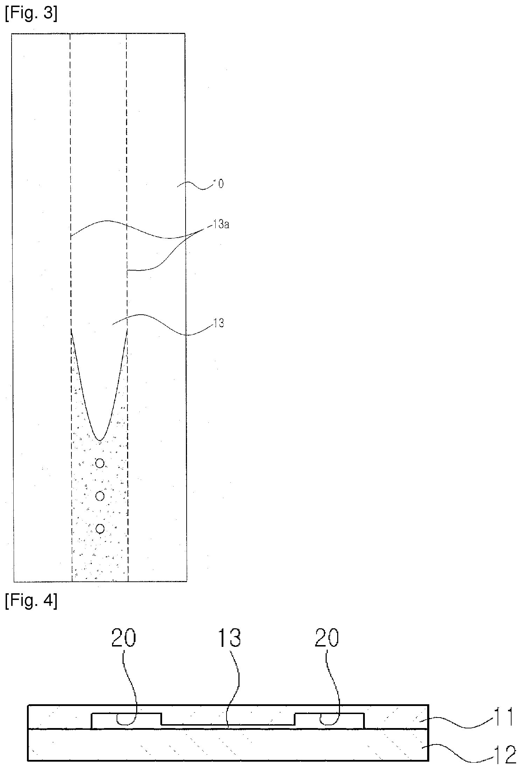

For example, a chip shown in FIGs. 1 and 2 is one example of a conventional chip. The chip includes a body 10 formed in such a manner that a first substrate 11 and a second substrate 12 are assembled with each other. A channel recess 13b having a predetermined width and depth is formed at the first substrate 11. In order to form a space to be filled with a sample, the channel recess 13b is formed at the first substrate 11 in such a manner that it extends in a longitudinal direction of the substrate while having a predetermined width and a predetermined depth. Therefore, when the first substrate 11 and the second substrate 12 are assembled with each other, the channel 13 has an airtight space. Also, a sample inlet 14, which extends to the outside of the channel so as to allow a sample to be injected into the channel 13, is formed at one end of the channel 13, and a sample outlet 15, which extends to the outside of the channel so as to allow the sample to be discharged, is formed at the other end of the channel. When sample fluid to be analyzed is injected into the channel 13, the injected sample fluid moves along the channel 13 toward the sample outlet 15. As shown in FIG. 3, when the sample fluid forms a pattern while moving, a difference of relative actions between the fluid, which passes though the channel, and upper, lower, left, and right inner walls 13a of the channel can cause a difference of capillary force, which is main driving force of movement of fluid in the microchannel. As a result, irregular pattern where a pattern is firstly formed along the left and right inner walls 13a is generated. Accordingly, many bubbles are generated in the fluid, thereby causing many problems in analyzing the sample fluid.

In a chip having a channel, which has a space for allowing fluids to flow and also has a sample inlet formed at one side of the channel and a sample outlet formed at the other side thereof, the inventors manufactured a chip having an expanding part, which has a sectional area larger than sectional areas of the left and right inner walls of the channel, formed at entire or a part of each left and right inner wall, and analyzed a pattern of movement of fluids. As a result, the inventors confirmed that the shape of movement of the fluids passing through the channel has a very regular and uniform pattern in the chip according to the present invention so that they completed the present invention.

### Disclosure of Invention

### Technical Problem

Therefore, the present invention has been made in view of the above-mentioned problems, and it is the object of the present invention to provide a fluid analysis chip, as described in claim 1.

Also, method for manufacturing a fluid analysis chip is described.

Also, a sample fluid analysis method using the fluid analysis chip is described.

### Technical Solution

In accordance with an aspect of the present invention, there is provided a chip including a sample inlet and a sample outlet communicating with the outside of the chip, this chip having a structure where the sample inlet and the sample outlet communicate with each other through a closed channel, wherein an expanding part is formed in a longitudinal direction of the channel in such a manner that a pair of inner walls corresponding to each other has a larger sectional area at a part or the whole thereof, so that fluid, which passes through the channel adjacent to the expanding part, moves while making contact with only another pair of inner walls of the channel, which correspond to each other. As such, in the fluid analysis chip according to the present invention, left and right inner walls of the closed channel are substantially expanded so that fluid passing through the channel can move while making contact with only upper and lower inner walls of the channel. As a result, relative action regarding movement of fluid depends on the upper and lower inner walls of the channel so that it is possible to obtain the uniform movement pattern of fluids.

Also, in manufacturing the fluid analysis chip according to the present invention, expanding recesses are formed at each entire part or each part of the left and right inner wall, respectively, in such a manner that they have a sectional area larger than a sectional area of each side inner wall of the channel. Since this doesn t need an accurate process of forming an edge of the channel, the fluid analysis chip is suitable for mass production.

### Advantageous Effects

As described above, a fluid analysis chip according to the present invention includes an expanded recess having an inner space, which has an expanded shape, and communicates with a channel. Therefore, a regular movement pattern of fluid passing through the channel is formed so that generation of bubbles is reduced, recurrence is secured, and it is easily performed to detect a signal from analytes in fluids. Also, in implementing a microchannel in a substrate, it is possible to manufacture the channel without concern of loss of each edge of the channel or deformation thereof. Therefore, it is easy to achieve mass production and quality control.

### Brief Description of the Drawings

The foregoing and other objects, features and advantages of the present invention will become more apparent from the following detailed description when taken in conjunction with the accompanying drawings in which:
FIG. 1 is a perspective view illustrating a conventional structure of a chip;
FIG. 2 is a sectional view of the chip shown in FIG. 1;
FIG. 3 is a plane view of the chip shown in FIG. 1;
FIG. 4 is a sectional view of an exemplary fluid analysis chip.
FIG. 5 is a plane view of the fluid analysis chip shown in FIG. 4 in which a movement pattern of fluids flowing in the chip is shown;
FIG. 6 is a sectional view of an exemplary fluid analysis chip. ;
FIG. 7 is a sectional view of a fluid analysis chip according to the present invention; and
FIG. 8 is a sectional view of an exemplary fluid analysis chip.

### Mode for the Invention

Hereinafter, fluid analysis chips will be described in detail with reference to the accompanying drawings. In description of the exemplary fluid analysis chips and chip of the present invention, structures and parts equal to them of a conventional chip designated by the same reference, and are described in detail with reference to FIG. 1.

As a term used in the present invention, a closed channel means a channel which is formed at an interior of a chip so that fluids can flow through the channel without exposure to outside of the chip, and has a shape similar to a pipe.

As a term used in the present invention, a channel inner wall means each surface of a channel, which limits a space allowing fluids flow therein.

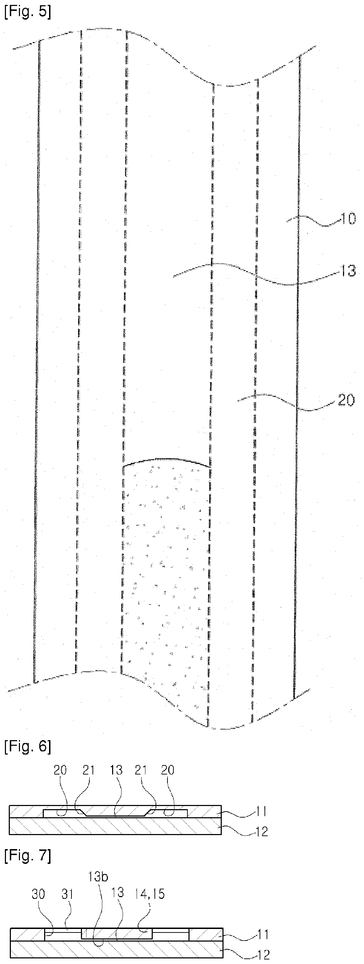

FIG. 4 is a sectional view of an exemplary fluid analysis chip, and FIG. 5 is a plane view of the fluid analysis chip.

As shown, this exemplary fluid analysis chip has a main body 10 formed in such a manner that a first substrate 11 and a second substrate 12 are assembled with each other. A channel recess 13b is formed at the first substrate 11 with a predetermined depth and a predetermined length, and extends in a longitudinal direction of the first substrate so as to form a space to be filled with simple fluids. Therefore, the channel 13 has an airtight space when the first substrate 11 and the second substrate 12 are assembled with each other, and a sample inlet 14 and a sample outlet 15 are formed at both ends of the channel 13, respectively.

Also, expanding recesses 20 are formed on the first substrate along left and right inner walls of the channel 13, respectively, while having each depth deeper than a depth of the channel 13, and extend along the left and right inner walls of the channel 13 in a longitudinal direction of the channel. When the first substrate is assembled with the second substrate 12, expanding parts as an expanding space communicating with both sides of a space formed by the channel 13 are formed.

In the exemplary fluid analysis chip shown in FIG. 5, when sample fluid including analytes is injected into the channel 13, the injected sample fluid moves toward the outlet 150 along the channel 13 and the expanding recesses 20 while making contact with the upper and lower inner walls of the channel so that a pattern is formed. At this time, relative action between the sample fluid and both side walls of the channel 13 decreases due to the expanding recesses 20 so that a uniform pattern of movement of the sample fluid is formed. Therefore, generation of bubbles decreases, recurrence is secured, and it is easily performed to detect signals from analytes.

A further exemplary fluid analysis chip is shown in FIG. 6. The fluid analysis chip includes a main body 10, which is formed in such a manner that a first substrate 11 and a second substrate 12 are assembled with each other. A channel recess 13b is formed at the first substrate 11 with a predetermined depth and a predetermined length, and extends in a longitudinal direction of first substrate so as to form a space to be filled with sample fluids. The channel 13 has an airtight space when the first substrate 11 and the second substrate 12 are assembled with each other, and a sample inlet 14 and a sample outlet 15 are formed at both ends of the channel 13, respectively. Also, as shown in FIG. 6, among both inner surfaces of each expanding recess, an inner surface bent toward the channel 13 is a slanting surface 21 having a depth becoming shallower toward the channel 13. The remaining structure and a formed sample pattern are equal to the exemplary fluid analysis chip of FIG. 4.

FIG. 7 illustrates the embodiment of the present invention. The fluid analysis chip according the present invention includes a main body 10, which is formed in such a manner that a first substrate 11 and a second substrate 12 are assembled with each other. A channel recess 13b is formed at the first substrate 11 with a predetermined depth and a predetermined length, and extends in a longitudinal direction of first substrate so as to form a space to be filled with sample fluids. The channel 13 has an airtight space when the first substrate 11 and the second substrate 12 are assembled with each other, and a sample inlet 14 and a sample outlet 15 are formed at both ends of the channel 13, respectively. As shown in FIG. 7, expanding holes 30, which extend through the first substrate 1 along both boundary surfaces of the channel 13, respectively, are formed at the first substrate 1 so as to form an expanding part. Each expanding hole 30 extends along both inner surfaces of the channel 13, and preferably has connecting parts 31 formed with each interval so as to prevent a product from breaking. The remaining structure and formed sample pattern are equal to the exemplary fluid analysis chip of FIG. 4.

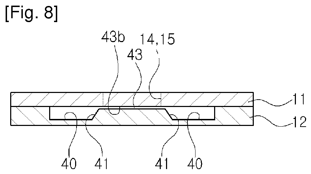

FIG. 8 illustrates a further exemplary fluid analysis chip. The fluid analysis chip includes a main body 10 formed in such a manner that a first substrate 11 and a second substrate 12 are assembled with each other. A channel recess 13b is formed at the first substrate 11 with a predetermined depth and a predetermined length and extends in a longitudinal direction of first substrate so as to form a space to be filled with sample fluids. The channel 13 has an airtight space when the first substrate 11 and the second substrate 12 are assembled with each other, and a sample inlet 14 and a sample outlet 15 are formed at both ends of the channel 13, respectively. Also, as shown in FIG. 8, among both inner surfaces of each expanding recess 40, an inner surface bent toward the channel 43 is a slanting surface 41 having a depth shallower toward the channel 43.

As such, the fluid analysis chip according to the present invention includes the expanding recesses 20 and 40, which are spaces expanding along both left and right inner surfaces, and the expanding holes 30. Therefore, when sample fluid is injected in the channel and moves due to the expanding recesses 20 and 40 the expanding holes 30, relative action between the left and right inner walls of the channel 13 and 43 and the sample fluid disappears or decreases so that the movement pattern of the sample fluid is uniformly formed along surfaces of the channel 13 and 43. Therefore, generation of bubbles in the fluid decreases, recurrence is secured, and it is possible to easily detect signals from analytes in the fluid.

The characteristic of each structure of the expanding recesses 20 and 40 and the expanding hole 30 makes it possible to prevent flatness of the channel 13 and 43 from decreasing due to contraction when the fluid analysis chip according to the present invention is formed by injection.

Moreover, the fluid analysis chips described can be manufactured in such a manner that at least two channel communicate, which sample inlets different from each other and sample outlets different from each other, respectively, are included in one chip. Also, it is possible to manufacture a fluid analysis chip having a structure where one sample inlet and one sample outlet communicate with each other through at least two channels.

## Claims

1. A fluid analysis chip comprising:
a main body (10) formed from assembling a first substrate (11) and a second substrate (12) with each other;
a channel recess (13b) arranged at one surface of the first substrate (11) and extending in a longitudinal direction of the first substrate (11);
a channel (13) formed from assembling the second substrate (12) on the surface of the first substrate (11) at which the channel recess (13b) is arranged;
a sample inlet (14) and a sample outlet (15) arranged at both ends of the channel (13), the sample inlet (14) and sample outlet (15) communicating with an outside of the chip; and
an expanding part (20) formed in a longitudinal direction of a whole of each of a left and a right inner wall of the channel (13) in such a manner that the expanding part (20) has a larger sectional area than sectional areas of the left and right inner wall of the channel (13) and communicates with the channel (13),
wherein the sample inlet (14) and the sample outlet (15) communicates with each other through the channel (13), so that fluid to be passed through the channel (13) adjacent to the expanding part (20) is moved while making contact with only an upper and a lower inner wall of the channel (13),
**characterized in that**,
the expanding part (20) is formed as expanding holes (30), which are formed at the first substrate (11) along the channel recess (13b), wherein each expanding hole (30) extends through the first substrate (11) along the channel (13) and has connecting parts (31) to prevent the chip from breaking.

## Patentansprüche

1. Chip zur Analyse von Flüssigkeiten, umfassend:
einen Hauptkörper (10), der durch Zusammenfügen eines ersten Substrats (11) und eines zweiten Substrats (12) gebildet worden ist;
eine kanalförmige Ausnehmung(13b), die an der Oberfläche des ersten Substrats (11) angeordnet ist und sich in einer Längsrichtung des ersten Substrats (11) erstreckt;
einen Kanal (13), der durch Anbringen des zweiten Substrats (12) auf der Oberfläche des ersten Substrats (11), an der die kanalförmige Ausnehmung(13b) angeordnet ist, gebildet worden ist;
einen Probeneinlass (14) und einen Probenauslass (15), die an beiden Enden des Kanals (13) angeordnet sind, wobei der Probeneinlass (14) und der Probenauslass (15) mit einer Außenseite des Chips kommunizieren; und
einen Erweiterungsbereich (20), der in einer Längsrichtung einer Gesamtheit jeweils einer linken und einer rechten Innenwand des Kanals (13) so ausgebildet ist, dass der Erweiterungsbereich (20) eine größere Querschnittfläche als die Querschnittflächen der linken und rechten Innenwand des Kanals (13) aufweist und mit dem Kanal (13) kommuniziert;
wobei der Probeneinlass (14) und der Probenauslass (15) miteinander über den Kanal (13) kommunizieren, so dass die durch den Kanal (13) zu leitende Flüssigkeit sich in der Nähe des Erweiterungsbereichs (20) bewegt, während sie nur mit einer oberen und einer unteren Innenwand des Kanals (13) in Kontakt steht,
**dadurch gekennzeichnet, dass** der Erweiterungsbereich (20) in Form von Erweiterungslöchern (30) ausgebildet ist, die am ersten Substrat (11) entlang der kanalförmigen Ausnehmung (13b) ausgebildet sind, wobei sich jedes Erweiterungsloch (30) durch das erste Substrat (11) entlang des Kanals (13) erstreckt und Verbindungsteile (31) aufweist, um einen Bruch des Chips zu verhindern.

## Revendications

1. Puce d'analyse de fluide comprenant :
un corps principal (10) formé en assemblant un premier substrat (11) et un second substrat (12) l'un avec l'autre ;
un renfoncement de canal (13b) prévu au niveau d'une surface du premier substrat (11) et s'étendant dans une direction longitudinale du premier substrat (11) ;
un canal (13) formé en assemblant le second substrat (12) sur la surface du premier substrat (11) au niveau duquel le renfoncement de canal (13b) est prévu ;
une admission d'échantillon (14) et une évacuation d'échantillon (15) prévues aux deux extrémités du canal (13), l'admission d'échantillon (14) et l'évacuation d'échantillon (15) communiquant avec un extérieur de la puce ; et
une partie d'extension (20) formée dans une direction longitudinale de l'intégralité de chacune d'une paroi intérieure gauche et d'une paroi intérieure droite du canal (13) de sorte que la partie d'extension (20) possède une section plus grande que les sections des parois intérieures de gauche et de droite du canal (13), et communique avec le canal (13),
dans laquelle l'admission d'échantillon (14) et l'évacuation d'échantillon (15) communiquent l'une avec l'autre par le biais du canal (13), de sorte que le fluide qui doit passer par le canal (13) adjacent à la partie d'extension (20) soit déplacé tout en entrant en contact uniquement avec une paroi intérieure supérieure et une paroi intérieure inférieure du canal (13),
**caractérisée en ce que**
la partie d'extension (20) est formée comme des orifices en extension (30), qui sont formés au niveau du premier substrat (11) le long du renfoncement de canal (13b), chaque orifice en extension (30) s'étendant dans le premier substrat (11) le long du canal (13) et ayant des parties de liaison (31) destinées à empêcher la puce de se casser.
